# EUROPÄISCHE PATENTSCHRIFT

(11) **EP 3 278 786 B1**
(45) Veröffentlichungstag und Bekanntmachung des Hinweises auf die Patenterteilung: **23.01.2019**
(21) Anmeldenummer: 16182622.7
(22) Anmeldetag: 03.08.2016
(51) Int. Cl.: A61K 6/083, C08F 22/14

(54) **DENTALMATERIALIEN AUF DER BASIS VON URETHANGRUPPENHALTIGEN VINYLCYCLOPROPAN-DERIVATEN**
DENTAL MATERIALS ON THE BASIS OF VINYLCYCLOPROPANE DERIVATIVES CONTAINING URETHANE GROUPS
MATERIAUX DENTAIRES A BASE DE DERIVES DE VINYLCYCLOPROPANE CONTENANT DES GROUPES URETHANE

(43) Veröffentlichungstag der Anmeldung: 07.02.2018
(73) Patentinhaber: Ivoclar Vivadent AG, 9494 Schaan (LI)
(72) Erfinder: Moszner, Norbert, 9493 Mauren (LI); Catel, Yohann, 9470 Buchs (CH); Fischer, Urs Karl, 9320 Arbon (CH); Tauscher, Sven, 9470 Buchs (CH)
(74) Vertreter: Uexküll & Stolberg

(56) Entgegenhaltungen:
- DE-A1- 19 714 320
- US-A1- 2012 010 322
- DATABASE WPI Week 201080 Thomson Scientific, London, GB; AN 2010-P22097 XP002766140, & JP 2010 260945 A (NISSAN CHEM IND LTD) 18. November 2010 (2010-11-18)

## Beschreibung

Die vorliegende Erfindung betrifft urethangruppenhaltige Vinylcyclopropane, die eine hohe Reaktivität bei der radikalischen Polymerisation aufweisen und die sich insbesondere zur Herstellung von Dentalwerkstoffen eignen, insbesondere von Zementen, Kompositen, Beschichtungsmaterialien, Bondings und Adhäsiven. Außerdem eigenen sich die Vinylcyclopropane zur stereolithographischen Herstellung von Formkörpern.

Die polymerisierbare organische Matrix von dentalen Harzen, Zementen oder Kompositen besteht vor allem aus einer Mischung von Monomeren, Initiator-Komponenten, Stabilisatoren und Pigmenten (J. Viohl, K. Dermann, D. Quast, S. Venz, Die Chemie zahnärztlicher Füllungskunststoffe, Carl Hanser Verlag, München-Wien1986, 21-27). Als Harze werden meistens Mischungen von Dimethacrylaten eingesetzt (vgl. A. Peutzfeldt, Resin composites in dentistry: the monomer systems, Eur. J. Oral. Sci. 105 (1997) 97-116; J. W. Nicolson, H. M. Anstice, The chemistry of modern dental filling materials, J. Chem Ed. 76 (1999) 1497-1501; J. W. Stansburry, Curing dental resins and composites by photopolymerization, J. Esthet. Dent., 12 (2000) 300-308; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 50 (2012) 4369-4402).

Ein Hauptnachteil der eingesetzten Methacrylate besteht darin, dass ihre Polymerisation von einer Volumenkontraktion, dem sog. Polymerisationsschrumpf begleitet wird. Bei Dentalmaterialien kann der Polymerisationsschrumpf u.a. zu nachteiligen Schrumpfungsspannungen und zur Randspaltbildung bei Füllungskompositen, zur verminderten Substrathaftung bei Befestigungskompositen oder Beschichtungsmaterialien sowie zur Beeinträchtigung der Dimensionsstabilität von Prothesenkunststoffen führen. In diesem Zusammenhang haben radikalisch polymerisierbare cyclische Monomere aufgrund des im Vergleich zu linearen Monomeren, wie z.B. Methacrylaten, deutlich geringeren Polymerisationsschrumpfes große Beachtung bei der Herstellung von Dentalwerkstoffen gefunden (vgl. R. K. Sadhir, R. M. Luck, Expanding Monomers, CRC Press, Boca Raton etc. 1992).

Vinylcyclopropane zeichnen sich gegenüber anderen bekannten ringöffnenden Monomeren, wie methylengruppenhaltigen Spiroorthocarbonaten (SOC), Spiroorthoestern (SOE) oder bicyclischen Orthoestern (BOE), dadurch aus, dass die Vinylcycloprpoyl (VCP)- Gruppe nicht feuchtigkeitsempfindlich ist und dass bei ihrer radikalischen Polymerisation Polymere mit hohen Molmassen erhalten werden, die in der Hauptkette nur hydrolytisch stabile C-C-Bindungen enthalten. (N. Moszner, F. Zeuner, T. Völkel, V. Rheinberger, Macromol. Chem. Phys. 200 (1999) 2173).

Aus der DE 198 12 888 A1 sind Vinylcyclopropanderivate und insbesondere Vinylcyclopropan(meth)acrylate bekannt, die sich mit Acrylaten und Methacrylaten copolymerisieren lassen.

Darüber hinaus sind Vinylcyclopropane mit mehreren polymerisationsfähigen Gruppen bekannt. F. Sanda, T. Takata, T. Endo, Macromolecules 27 (1994) 3986, beschreiben 1-Vinyl-5,7-dioxaspiro[2.5]octan-6-on, ein Hybridmonomer, das eine Vinylcyclopropan- und eine cyclische Carbonat-Gruppe enthält, und T. Okazaki, F. Sanda, T. Endo, Macromolecules 28 (1995) 6026, 1,10-Bis(vinyl)-4,8,12,15-tetraoxatrispiro[2.2.2.2.2.2]pentadecan, ein Monomer, bei dem zwei Vinylcyclopropangruppen über eine hydrolyseempfindliche Spiroacetal-Einheit miteinander verbunden sind. Diese Verbindungen weisen im Vergleich zu monofunktionellen Vinylcyclopropanen keine verbesserte radikalische Copolymerisationsfähigkeit mit (Meth)acrylverbindungen auf.

Die EP 0 798 286 A1 betrifft multifunktionelle Vinylcyclopropanderivate mit zwei bis sechs Vinylcyclopropangruppen, die die Herstellung vernetzter Polymere erlauben.

Am Beispiel der radikalischen Copolymerisation von 1,1-Bis-(ethoxycarbonyl)-2-vinylcyclopropan mit Methylmethacrylat (MMA) konnte gezeigt werden (F. Sanda, T. Takata, T. Endo, Macromolecules, 27 (1994) 3982), dass sich Vinylcyclopropane im Vergleich zu Methacrylaten durch ein geringeres radikalisches Polymerisationsvermögen auszeichnen, was deren praktischen Einsatz deutlich einschränkt. Besonders nachteilig ist, dass die bekannten, einfach zugänglichen 1,1-Bis-(alkoxycarbonyl)-2-vinylcyclopropane eine geringe Photopolymerisationsaktivität aufweisen.

Die EP 1 413 569 A1 offenbart Dentalwerkstoffe auf der Basis von bicyclischen Cyclopropanderivaten, wie z.B. 2-[Bicyclo-[3.1.0]hex-1-yl]-acrylsäuremethylester, die eine verbesserte Reaktivität bei der radikalischen Polymerisation zeigen (N. Moszner, F. Zeuner, U.K. Fischer, V. Rheinberger, A. de Meijere, V. Bagutski, Macromol. Rapid. Commun. 24 (2003) 269). Diese reaktiveren monofunktionellen bicyclischen Cyclopropylacrylate sind allerding nur sehr schwierig zugänglich.

Das Dokument DE19714320 beschreibt urethangruppenhaltige VinylcyclopropanDerivate, die Silan-terminiert sind und für Dentalmaterialien verwendet werden.

Das Dokument US2012010322 beschreibt polymerisierbare Monomere, die eine Vinylcyclopropan-Gruppe enthalten und eine Urethangruppe enthalten können.

Der Erfindung liegt die Aufgabe zugrunde, radikalisch polymerisierbare Monomere bereitzustellen, die bei der radikalischen Polymerisation nur wenig schrumpfen und die eine hohe radikalische Polymerisationsreaktivität aufweisen, insbesondere bei der Photopolymerisation. Außerdem sollen die Monomere synthetisch gut zugänglich sein und sich zur Herstellung von Dentalwerkstoffen wie Adhäsiven, Zementen oder Kompositen sowie zur Herstellung von Beschichtungen und Formkörpern eignen, beispielsweise zur stereolithographischen Herstellung von Formkörpern.

Diese Aufgabe wird durch urethangruppenhaltige Vinylcyclopropane entsprechend der allgemeinen Formeln I gelöst: 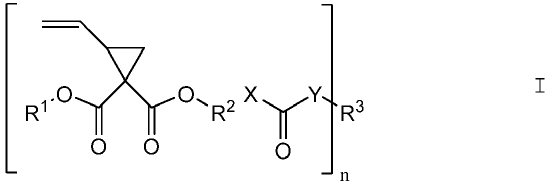 in der
- X, Y: jeweils O oder NH, wobei X und Y nicht dieselbe Bedeutung haben können,
- R¹: ein verzweigter oder vorzugsweise linearer C₁-C₄-Alkylrest,
- R²: ein verzweigter oder vorzugsweise linearer C₂-C₄-Alkylrest,
- R³: ein aliphatischer linearer oder verzweigter C₁-C₂₀-Kohlenwasserstoffrest, ein aromatischer C₆-C₁₄-Rest, ein aromatischer oder nicht aromatischer heterocyclischer Rest, der 4 bis 12 Kohlenstoffatome und 1 bis 2 Hetero-atome enthält, die aus N- oder O-Atomen ausgewählt sind, ein alicyclischer C₆-C₁₂-Kohlenwasserstoffrest ist, wobei es sich bei den cyclischen Resten in allen Fällen um mono- oder polycyclische Gruppen handeln kann, oder ein aliphatisch-aromatischer C₇-C₂₀-Rest,
- n: 1 oder 2.
Verbindungen der Formel I sind besonders bevorzugt, in denen die Variablen die folgenden Bedeutungen haben:
- X, Y: jeweils O oder NH, wobei X und Y nicht dieselbe Bedeutung haben können,
- R¹: ein Methyl- oder Ethyl-Rest,
- R²: ein linearer C₂-C₄-Alkylenrest,
- R³: ein aliphatischer linearer oder verzweigter C₁-C₁₀-Kohlenwasserstoffrest, ein aromatischer C₆-C₁₄-Rest, ein alicyclischer C₆-C₁₂-Kohlenwasserstoffrest ist, wobei es sich bei den cyclischen Resten in allen Fällen um mono- oder polycyclische Gruppen handeln kann, oder ein aliphatisch-aromatischer C₇-C₁₅-Rest,
- n: 1 oder 2.

Die Vinylcyclopropane der Formel I zeichnen sich durch einen geringen Schrumpf und eine hohe Reaktivität bei der radikalischen Polymerisation aus. Gegenstand der vorliegenden Erfindung ist auch die Verwendung von urethangruppenhaltigen Vinylcyclopropanen der Formel I zur Herstellung von Dentalwerkstoffen, insbesondere von dentalen Zementen, Kompositen, Beschichtungsmaterialien, Bondings und Adhäsiven, sowie zur Herstellung von Werkstoffen zur stereolithographischen Herstellung von Formkörpern. Ein weiterer Gegenstand der Erfindung sind Formkörper, Polymere und Copolymere, die durch Homo- oder Copolymerisation der urethangruppenhaltigen Vinyl-cyclopropane der Formel I erhältlich sind.

Die urethangruppenhaltigen monofunktionellen oder multifunktionellen Vinylcyclopropane der allgemeinen Formeln I können einfach hergestellt werden. Die Synthese von 1,1-di(alkoxycarbonyl)-substituierten 2-Vinylcyclopropane kann nach bekannten Verfahren (vgl. US 4,713,478 und US 4,713,479) z.B. durch Umsetzung von trans-1,4-Dihalogenbut-2-enen mit entsprechenden Malonsäureestern erfolgen:

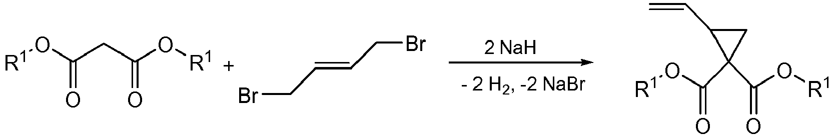

### Beispielsweise:

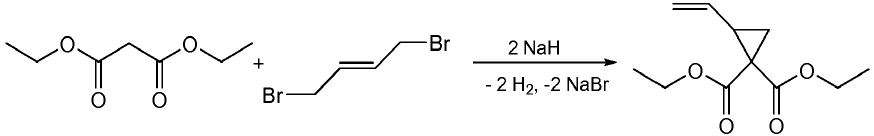

Aus den 1,1-di(alkoxycarbonyl)-substituierten 2-Vinylcyclopropanen lassen sich durch partielle alkalische Hydrolyse 2-Vinylcyclopropan-1-alkoxycarbonyl-1-carbonsäuren herstellen:

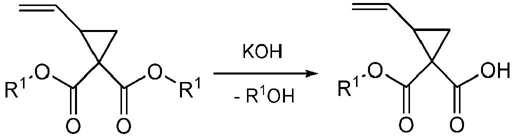

### Beispielsweise:

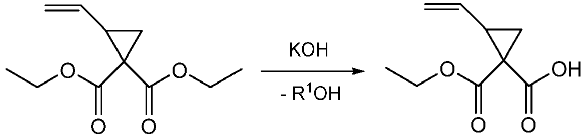

Aus den 2-Vinylcyclopropan-1-alkoxycarbonyl-1-carbonsäuren lassen sich durch Umsetzung mit Diolen in Gegenwart von Dicyclohexylcarbodiimid (DCC) und 4-Dimethylaminopyridin (DMAP) entsprechende Hydroxyalkyl-Derivate synthetisieren:

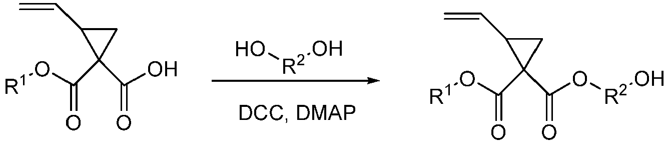

### Beispielsweise:

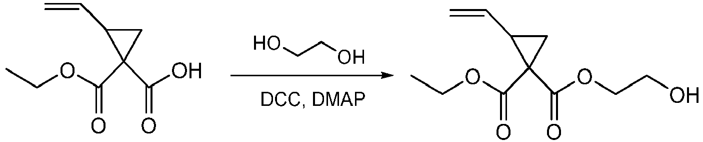

Die Reaktion zwischen solchen Hydroxyalkyl-Derivaten und mono- oder multifunktionellen Isocyanaten ergibt die Urethan-Vinylcyclopropane:

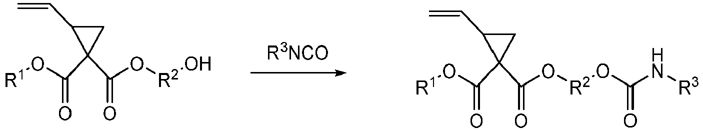

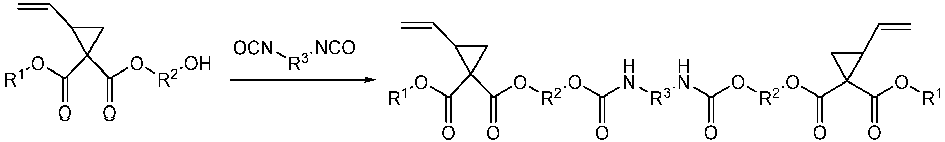

### Beispielsweise:

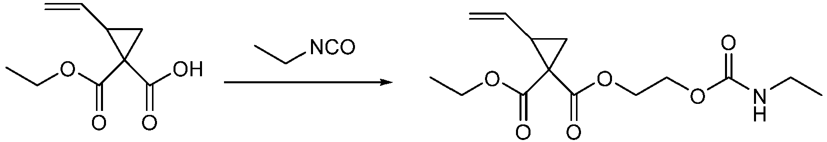

Aus der 2-Vinylcyclopropan-1,1-dicarbonsäure lassen sich durch Umsetzung mit Diolen in Gegenwart von DCC und DMAP entsprechende Didyroxyalkyl-Derivate herstellen. Solche Verbindungen könnten anschließend mit Isocyanaten zu den entsprechenden Diurethanen reagieren:

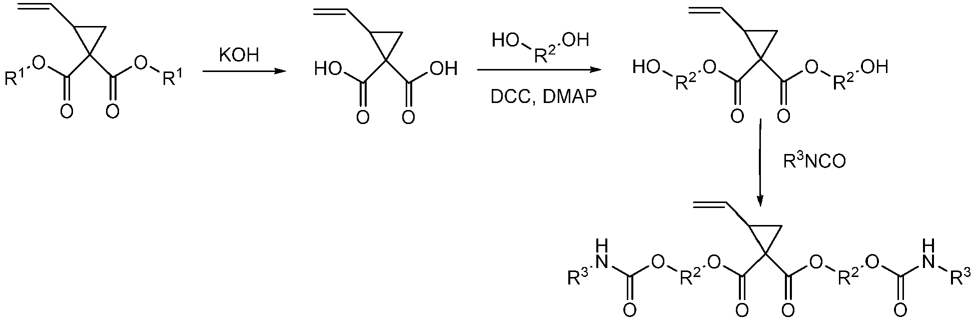

Aus den 2-Vinylcyclopropan-1-alkoxycarbonyl-1-carbonsäuren lassen sich durch Umsetzung mit N-Boc-Hydroxyalkylaminen (BOC = tert-Butyloxycarbonyl) in Gegenwart von DCC und DMAP entsprechende BOC-geschützte Amine synthetisieren. Eine Entschützung mit Trifluoressigsäure (TFA) liefert die entsprechenden Amine:

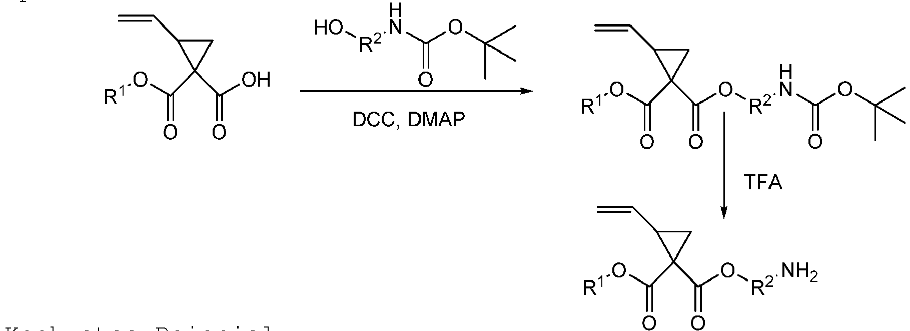

### Konkretes Beispiel:

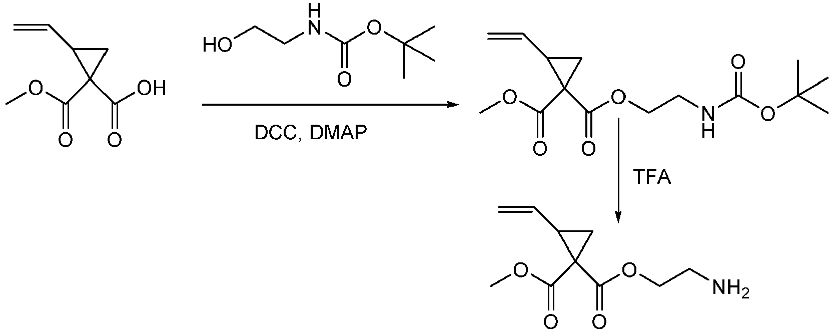

Diese Amine können dann durch die Reaktion mit Dialkylcarbonaten zu erfindungsgemäßen Urethan-Derivaten umgesetzt werden.

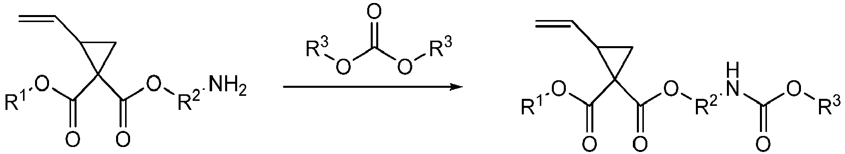

### Beispielsweise:

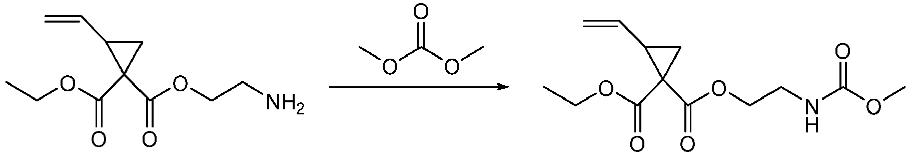

Eine weitere Möglichkeit zur Synthese der urethangruppenhaltigen mono- oder multifunktionellen Vinylcyclopropane der allgemeinen Formeln I besteht in der Reaktion von Vinylcyclopropan-Aminen mit Alkylchloroformaten. Die Alkylchloroformate können aus dem Bis(trichloromethyl)carbonat und dem entsprechenden Alkohol synthetisiert werden:

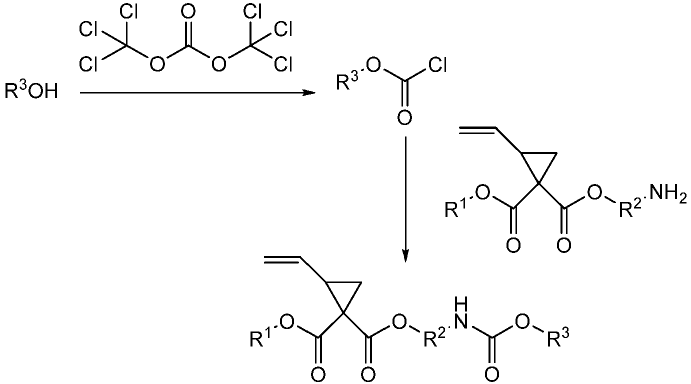

Mit dieser Methode können multifunktionelle Vinylcyclopropane auf die folgende Weise hergestellt werden:

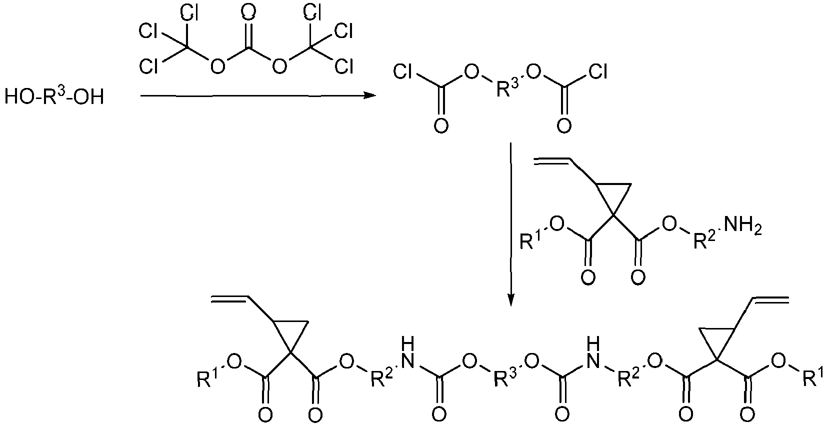

### Konkretes Beispiel:

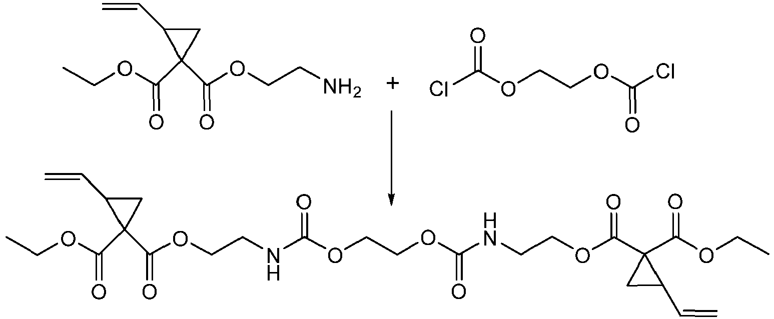

Bevorzugte Beispiele für urethangruppenhaltige monofunktionelle oder multifunktionelle Vinylcyclopropane sind:

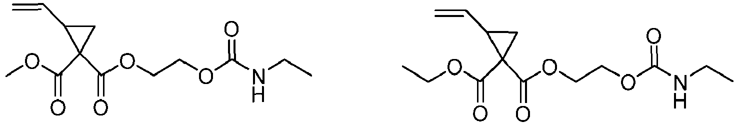

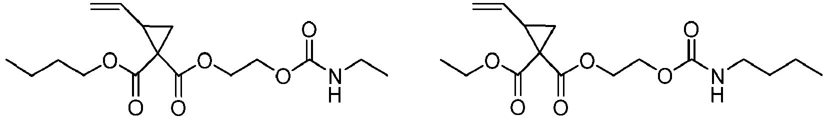

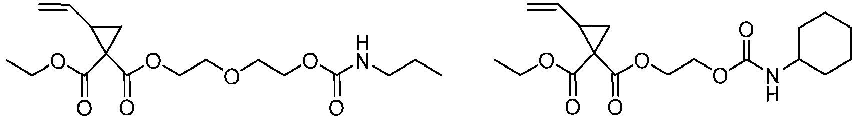

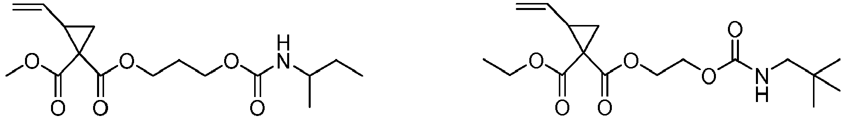

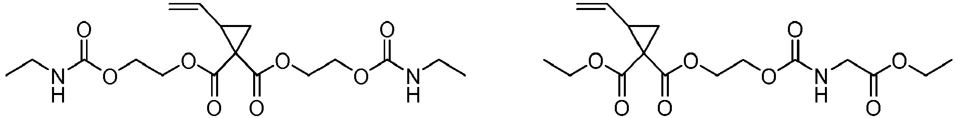

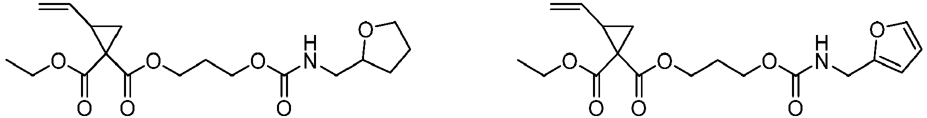

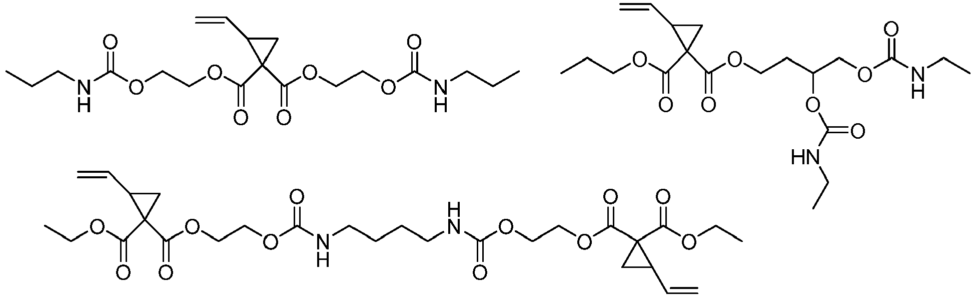

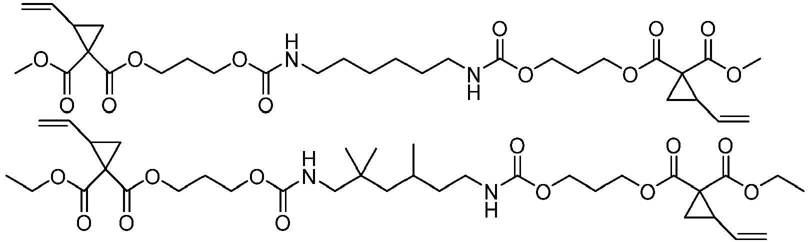

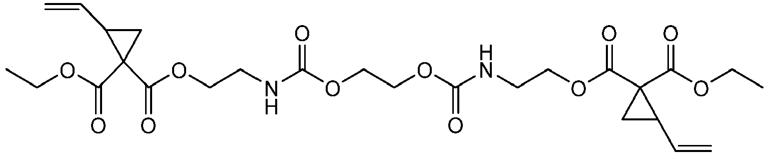

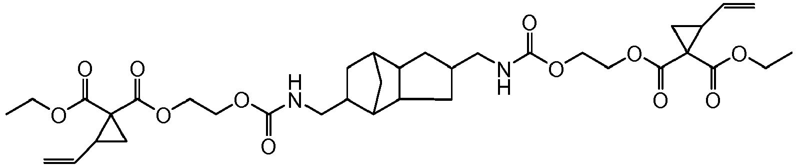

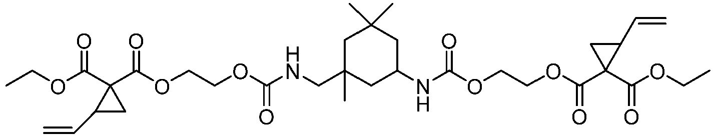

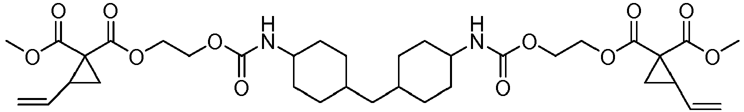

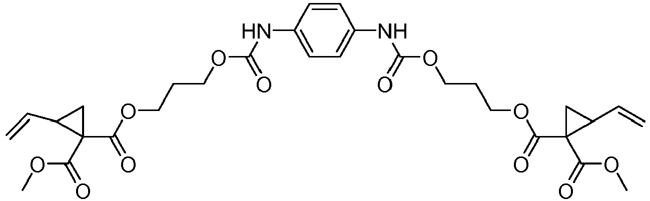

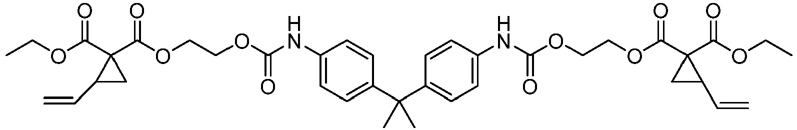

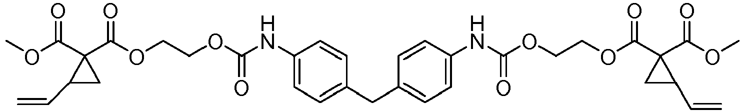

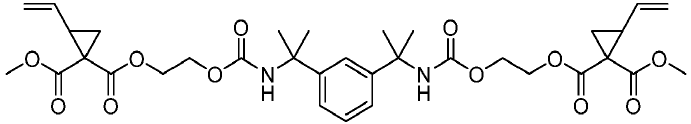

Die polymerisationsfähigen urethangruppenhaltigen Vinylcyclopropane der allgemeinen Formel I sind meist flüssig und zeigen überraschenderweise eine im Vergleich zu 1,1-Bis-(alkoxycarbonyl)-2-vinylcyclopropanen deutlich verbesserte radikalische Polymerisierbarkeit, insbesondere bei der Photopolymerisation, und im Vergleich zu nicht cyclischen Methacrylaten eine geringere Polymerisationsschrumpfung. Damit lassen sich Dentalwerkstoffe wie Befestigungszemente oder Füllungskomposite mit ebenfalls verringertem Polymerisationsschrumpf herstellen.

Die erfindungsgemäßen (Dental-)Werkstoffe enthalten vorzugsweise 2 bis 95 Gew.-%, besonders bevorzugt 5 bis 85 Gew.-%, ganz besonders bevorzugt 5 bis 60 Gew.-% und insbesondere 10 bis 60 Gew.-% Vinylcyclopropan(e) der allgemeinen Formel I, jeweils bezogen auf die Gesamtmasse des Werkstoffs.

Neben einem oder mehreren Vinylcyclopropanen der allgemeinen Formel I enthalten die Werkstoffe vorzugsweise mindestens ein weiteres radikalisch polymerisierbares Monomer. Als weitere radikalisch polymerisierbare Monomere sind andere Vinylcyclopropane wie 1,1-Di(ethoxycarbonyl)- oder 1,1-Di(methoxycarbonyl)-2-vinylcyclopropan, Bis(2-vinylcyclpropan-1-carbonsäureethylester-1-carbonamido)-2,2-dimethyl-4-methylhexan, 1,8-Bis(2-vinylcyclopropan-1-carbonsäureethylester-1-carbonyloxy)-3,6-dioxaoctan (VCP 5) oder die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin bevorzugt, wie Bis-(2-vinyl-1,1-di-carbonsäuremonoethylester)resorcinylester (VCP 4), und insbesondere die in N. Moszner, F. Zeuner, V. Rheinberger, Macromol. Rapid Commun. 18 (1997) 775-780, und N. Moszner, F. Zeuner, T. Völkel, U. K. Fischer, V. Rheinberger, J. Appl. Polym. Sci. 72 (1999) 1775-1782 beschriebenen 2-Vinylcyclopropane. Weiter bevorzugt sind die in EP 1 413 569 A1 offenbarten bicyclischen Cyclopropanderivate, insbesondere 2-(Bi-cyclo[3.1.0]hex-1-yl)acrylsäuremethyl- oder -ethylester oder deren Disubstitutionsprodukte in 3-Stellung wie (3,3-Bis-(ethoxycarbonyl)bicyclo[3.1.0]hex-1-yl)acrylsäuremethyl- oder -ethylester.

Ebenso bevorzugt sind die in der EP 1 688 125 A1 offenbarten Cyclopropylacrylate, insbesondere {3,3-Bis(ethoxycarbonyl)-bicylo[3.1.0]hexa-1-yl}acrylsäuremethylester und 2-(3-Acetyl-3-ethoxycarbonyl-bicyclo[3.1.0]hexa-1-yl}acrylsäuremethylester.

Dentalwerkstoffe die als Comonomer mindestens ein monofunktionelles Vinylcyclopropan mit der allgemeinen Formeln II enthalten werden auch offenbart: 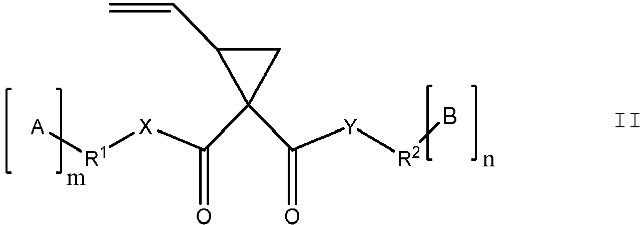 in der
- A, B: unabhängig voneinander jeweils OH oder COOH sind,
- X, Y: unabhängig voneinander jeweils O oder NH sind,
- R¹, R²: unabhängig voneinander jeweils H oder ein aliphatischer linearer oder verzweigter C₁-C₁₀-Kohlenwasserstoffrest sind, der durch O, S oder eine Estergruppe unterbrochen sein kann, ein alicyclischer oder aromatischer C₆-C₁₄-Rest oder ein aromatischer oder nicht aromatischer heterocyclischer Rest, der 4 bis 14 Kohlenstoffatome und 1 bis 6 Heteroatome enthalten kann, die aus N-, O-, und/oder S-Atomen ausgewählt sind, und
- n, m: unabhängig voneinander jeweils 0, 1 oder 2 sind.

Wenn X = Y =0, R¹ ≠ H und R² ≠ H, dann ist n+m≥1. Wenn m = 0 und R¹ ≠ H, wird die freie Valenz an R¹ durch H abgesättigt, und wenn n = 0 und R² ≠ H, wird dementsprechend die freie Valenz an R² durch H abgesättigt.

Die Verbindungen der Formel II zeichnen sich dadurch aus, dass sie über Gruppen (CO-NH, OH, COOH) verfügen, die zur Ausbildung von Wasserstoffbrückenbindungen geeignet sind. Die Vinylcyclopropane der Formel II haben eine geringe Viskosität und eignen sich daher besonders als Verdünnermonomere für dickflüssige Monomere.

Verbindungen der Formel II, in denen A und B dieselbe Bedeutung haben, sind bevorzugt. Ebenso sind solche Verbindungen bevorzugt, in denen X und Y dieselbe Bedeutung haben. R¹ und R² können gleich oder vorzugsweise verschieden sein. Die Reste R¹ und/oder R² der Formel II können durch eine oder mehrere der genannten Atome und Gruppen unterbrochen sein. Bevorzugt sich Reste, die durch eine Gruppe oder ein Atom oder besonders bevorzugt nicht unterbrochen sind.

Bei den genannten cyclischen Resten der Formel II kann es sich um mono- oder polycyclische Gruppen handeln. R¹ und/oder R² können auch durch eine Kombination der genannten Reste gebildet werden, beispielsweise durch eine Kombination von einer oder mehreren aliphatischen und einer oder mehreren aromatischen Gruppen, z.B. ein aliphatisch-aromatischer C₇-C₁₄-Rest. Besonders bevorzugt sind Reste, die eine Tricyclodecangruppe (TCD) enthalten.

Verbindungen der Formel II sind bevorzugt, in denen die Variablen die folgenden Bedeutungen haben:
- A: OH,
- m: 0, 1 oder 2,
- R¹: H oder ein verzweigter oder vorzugsweise linearer C₁-C₆-Kohlenwasserstoffrest, der durch O unterbrochen sein kann, wobei Reste, die nicht durch O unterbrochen sind, bevorzugt sind, wobei R¹ vorzugsweise nicht H ist, wenn X = O und m = 0,
- X: O oder NH, wobei X vorzugsweise NH ist, wenn m = 0 und R¹ ≠ H,
- n: 0,
- R²: ein verzweigter oder vorzugsweise linearer C₁-C₃-Alkylrest,
- Y: O;
oder bevorzugt
- B: OH,

- n: 0, 1 oder 2,
- R²: H oder ein verzweigter oder vorzugsweise linearer C₁-C₆-Kohlenwasserstoffrest, der durch O unterbrochen sein kann, wobei Reste, die nicht durch O unterbrochen sind, bevorzugt sind, wobei R² vorzugsweise nicht H ist, wenn Y = O und n = 0,
- Y: O oder NH, wobei Y vorzugsweise NH ist, wenn n = 0 und R² ≠ H,
- m: 0,
- R¹: ein verzweigter oder vorzugsweise linearer C₁-C₃-Alkylrest
- X: O.

Besonders bevorzugt sind Verbindungen der Formel II, in denen die Variablen die folgenden Bedeutungen haben:
- A: OH,
- m: 0 oder 1,
- R¹: H oder ein linearer C₁-C₆-Kohlenwasserstoffrest, wobei R¹ vorzugsweise nicht H ist, wenn X = O und m = 0,
- X: O oder NH, wobei X NH ist, wenn m = 0 und R¹ ≠ H,
- n: 0,
- R²: ein linearer C₁-C₃-Alkylrest
- Y: O;
oder bevorzugt
- B: OH,
- n: 0 oder 1,
- R²: H oder ein linearer C₁-C₆-Kohlenwasserstoffrest, wobei R² vorzugsweise nicht H ist, wenn Y = O und n = 0,
- Y: O oder NH, wobei Y NH ist, wenn n = 0 und R² ≠ H,
- m: 0,
- R¹: ein linearer C₁-C₃-Alkylrest,
- X: O.

Außerdem können die Dentalwerkstoffe auch radikalisch polymerisierbare mono- oder polyfunktionelle (Meth)acrylsäurederivate enthalten. Unter monofunktionellen Monomeren werden Verbindungen mit einer, unter multifunktionellen Monomeren Verbindungen mit zwei oder mehr, vorzugsweise 2 bis 4 radikalisch polymerisierbaren Gruppen verstanden.

Bevorzugte mono- oder multifunktionelle Methacrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)-acrylat, Bis-GMA (ein Additionsprodukt aus Methacrylsäure und Bisphenol-A-diglycidylether), ethoxy- oder propoxyliertes Bisphenol-A-Dimethacrylat, wie z.B. das Bisphenol-A-Dimethacrylat 2-[4-(3-Methacryloyloxyethoxyethyl)phenyl]-2-[4-(3-methacryloyloxyethyl)phenyl]-propan) (SR-348c) mit 3 Ethoxygruppen oder 2,2-Bis[4-(2-(meth)acryloxypropoxy)-phenyl]propan, UDMA (ein Additionsprodukt aus 2-Hydroxyethylmethacrylat (HEMA) und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Di-, Tri- oder Tetraethylenglycoldi(meth)acrylat, Trimethylolpropantri-(meth)acrylat, Pentaerythrittetra(meth)acrylat, sowie Glycerindi- und trimethacrylat, 1,4-Butandioldi(meth)acrylat, 1,10-Decandioldi(meth)acrylat (D₃MA) oder 1,12-Dodecandiol-di(meth)acrylat.

Darüber hinaus können die Dentalwerkstoffe vorteilhaft auch radikalisch polymerisierbare, säuregruppenhaltige Monomere enthalten, wie z.B. polymerisationsfähige Carbonsäuren, Phosphonsäuren und Phosphorsäureester. Bevorzugte Beispiele für polymerisationsfähige Carbonsäuremonomere sind Maleinsäure, 2-(Hydroxymethyl)acrylsäure und 4-(Meth)acryloyloxyethyltrimellitsäureanhydrid. Bevorzugte Beispiele für geeignete Phosphonsäuremonomere sind 2-Methacryloyloxyethylphosphonsäure, 2-Methacrylamidoethylphosphonsäure, 2-[4-(Dihydroxyphosphoryl)-2-oxa-butyl]-acrylsäureethyl- oder -2,4,6-tri-methylphenylester. Bevorzugte Beispiele für geeignete acide polymerisationsfähige Phosphorsäureester sind 2-Methacryloyloxypropyldihydrogenphosphat, 2-Methacryloyloxyethyldihydrogenphosphat, 2-Methacryloyloxyethylphenyl-hydrogenphosphat, Dipentaerythritolpentamethacryloyloxyphosphat, 10-Methacryloyloxydecyldihydrogenphosphat und 6-(Methacrylamido)hexyldihydrogenphosphat. Acide Monomere dienen primär zur Verbesserung der Haftung der Werkstoffe an Dentin und/oder Zahnschmelz. Die Menge an aciden Monomeren liegt vorzugsweise im Bereich von 0 bis 20 Gew.-%, bevorzugt 0 bis 15 Gew.-% und besonders bevorzugt 0 bis 10 Gew.-%, bezogen auf die Gesamtmasse des Dentalwerkstoffs.

Besonders bevorzugt sind Werkstoffe, die mindestens ein multifunktionelles radikalischen Monomer, d.h. ein Vinylcyclopropan der Formel I und/oder ein anderes multifunktionelles radikalisch polymerisierbares Monomer enthalten.

Die erfindungsgemäßen Dentalwerkstoffe enthalten vorzugsweise weiterhin einen Initiator für die radikalische Polymerisation. Zur Initiierung der radikalischen Photopolymerisation sind Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil bevorzugt. Besonders bevorzugt werden Campherchinon und 2,2-Dimethoxy-2-phenyl-acetophenon und ganz besonders bevorzugt α-Diketone in Kombination mit Aminen als Reduktionsmittel, wie z.B. 4-(Dimethylamino)benzoesäureethylester, N,N-Dimethylaminoethylmethacrylat, N,N-Dimethyl-sym.-xylidin oder Triethanolamin, verwendet. Besonders geeignet sind auch Norrish-Typ-I-Photoinitiatoren, vor allem Acyl- der Bisacylphosphinoxide, wie beispielsweise die kommerziell erhältlichen Verbindungen 2,4,6-Trimethylbenzoyldiphenylphosphinoxid und Bis(2,4,6-trimethylbenzoyl)phenylphosphinoxid. Besonders geeignet sind auch Monoacyltrialkyl-, Diacyldialkylgermanium-, Triacylalkyl- sowie Tetraacylgermanium-Verbindungen, wie z.B. Benzoyltrimethylgermanium, Dibenzoyldiethylgermanium oder Bis(4-methoxybenzoyl)diethylgermanium sowie Tetrabenzoylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester oder Tetrabenzoylgermanium. Es können vorteilhaft auch Initiatorkombinationen eingesetzt werden, die zusätzlich aromatische Diaryliodonium- oder Triarylsulfoniumsalze enthalten, beispielsweise die kommerziell erhältlichen Verbindungen 4-Octyloxyphenyl-phenyl-iodoniumhexafluoroantimonat oder Isopropylphenyl-methylphenyl-iodoniumtetrakis-(pentafluorophenyl)borat.

Als Initiatoren für eine bei Raumtemperatur durchgeführte Polymerisation werden vorzugsweise Redox-Initiatorkombinationen, wie z.B. Kombinationen von Benzoylperoxid mit N,N-Dimethyl-sym.-xylidin oder N,N-Dimethyl-p-toluidin, verwendet. Darüber hinaus sind auch Redoxsysteme bestehend aus Peroxiden bzw. Hydroperoxiden und solchen Reduktionsmitteln, wie z.B. Ascorbinsäure, Barbiturate, Thioharnstoffe oder Sulfinsäuren, besonders geeignet. Außerdem lassen sich als Redoxkatalysatoren Verbindungen von Übergangsmetallen, die mindestens zwei stabile Wertigkeitsstufen zeigen, einsetzen. Das sind vor allem Verbindungen der Elemente Kupfer, Eisen, Vanadium, Nickel oder Kobalt, wobei besonders bevorzugt Kupferverbindungen sind und diese bevorzugt als gut organolösliche Verbindungen, wie z.B. als Acetylacetonat, Naphthenat oder 2-Ethyl-hexanoat, eingesetzt werden.

Dentalwerkstoffe, die mindestens einen Photoinitiator enthalten, sind erfindungsgemäß bevorzugt. Die Werkstoffe können zusätzlich weitere Initiatoren enthalten.

Weiterhin enthalten die erfindungsgemäß eingesetzten Zusammensetzungen vorzugsweise auch mindestens einen organischen oder besonders bevorzugt anorganischen partikulären Füllstoff, beispielsweise zur Verbesserung der mechanischen Eigenschaften oder zur Einstellung der Viskosität. Bevorzugte anorganische partikuläre Füllstoffe sind amorphe kugelförmige Materialien auf der Basis von Oxiden, wie SiO₂, ZrO₂ und TiO₂ bzw. Mischoxiden aus SiO₂, ZrO₂ und/oder TiO₂, nanopartikuläre oder mikrofeine Füllstoffe, wie pyrogene Kieselsäure oder Fällungskieselsäure (gewichtsmittlere Partikelgröße von 10-1000 nm), sowie Minifüllstoffe, wie Quarz-, Glaskeramik- oder Glaspulver mit einer gewichtsmittlere Partikelgröße von 0,01 bis 1 pm. Weitere bevorzugte Füllstoffe sind röntgenopake Füllstoffe, wie Ytterbiumtrifluorid, nanopartikuläres Tantal(V)-oxid, Bariumsulfat, Mischoxide von SiO₂ mit Ytterbium(III)-oxid oder Tantal(V)-oxid (gewichtsmittlere Partikelgröße von 10-1000 nm) und röntgenopake Glaspulver, z.B. Barium- oder Strontiumaluminiumsilikatgläser (gewichtsmittlere Partikelgröße von 0,2-10 pm).

Zur Verbesserung des Verbundes zwischen den Füllstoffpartikeln und der vernetzten Polymerisationsmatrix können die Füllstoffpartikel mit geeigneten Kupplungsreagenzien oberflächenmodifiziert sein. Für SiO₂-basierende Füllstoffe wie SiO₂, Quarz-, Glaskeramik- oder Glaspulver eignen sich besonders Trialkoxysilanen, wie z.B. 3-Methacryloyloxypropyltrimethoxysilan. Bevorzugt sind Trialkoxysilane, die Vinylcyclopropangruppen enthalten, ganz besonders die in der EP 0 867 444 A2 beschriebenen Vinylcyclopropansilane, wobei hier insbesondere die 1-Methoyxy- oder 1-Ethoxycarbonyl-1-[(3-trimethoxy- oder 3-triethoxysilyl)propylaminocarbonyl)]-2-vinylcyclopropane zu nennen sind. Konkret sind Vinylcycloproansilane mit den folgenden Strukturen besonders geeignet:

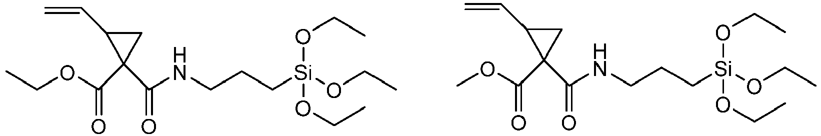

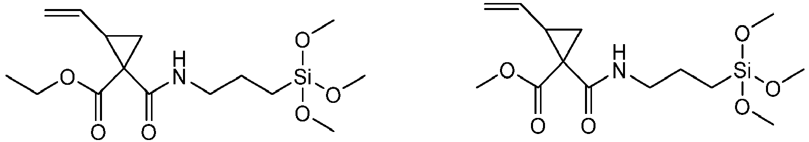

Zur Oberflächenmodifizierung von nicht-silikatischen Füllstoffen, z.B. von ZrO₂ oder TiO₂, können auch funktionalisierte saure Phosphate, wie z.B. 10-Methacryloyloxydihydrogenphospaphat eingesetzt werden.

Gegebenenfalls können die erfindungsgemäß eingesetzten Zusammensetzungen eine oder mehrere weitere Additive enthalten, vor allem Lösungsmittel, vorzugsweise Wasser, Ethanol oder eine Mischung davon, sowie Stabilisatoren, wie z.B. Polymerisationsstabilisatoren, Aromastoffe, Farbmittel, mikrobiocide Wirkstoffe, fluoridionenabgebende Additive, optische Aufheller, Weichmacher und/oder UV-Absorber.

Die erfindungsgemäßen Werkstoffe eignen sich besonders als dentale Zemente, Füllungsmaterialien, Beschichtungs- oder Verblendmaterialien.

Dabei sind erfindungsgemäß solche Dentalmaterialien bevorzugt, die die folgenden Komponenten enthalten:
a) 2 bis 95 Gew.-%, bevorzugt 2 bis 90 Gew.-% und besonders bevorzugt 10 bis 85 Gew.-% mindestens eines Vinylcyclopropans der allgemeinen Formel I,
b) 0,01 bis 5 Gew.-%, bevorzugt 0,1 bis 3,0 Gew.-%, besonders bevorzugt 0,2 bis 2 Gew.-% mindestens eines Initiators für die radikalische Polymerisation, vorzugsweise einen Photoinitiator,
c) 0 bis 80 Gew.-%, bevorzugt 0 bis 60 Gew.-% und besonders bevorzugt 0 bis 50 Gew.-% andere(s) Monomer(en), und ggf.
d) 0 bis 85 Gew.-%, bevorzugt 0 bis 80 Gew.-% Füllstoff(e).

Der Füllstoffgehalt richtet sich maßgeblich nach der gewünschten Anwendung des Dentalwerkstoffs. Dentalwerkstoffe zur Verwendung als Beschichtungsmaterial enthalten besonders bevorzugt 0 bis 40 Gew.-%, Dentalwerkstoffe zur Verwendung als Zement vorzugsweise 10-70 Gew.-% und Dentalwerkstoffe zur Verwendung Füllungsmaterial (Füllungskomposit) vorzugsweise 10-85 Gew.-% Füllstoff(e).

Dentalwerkstoffe zur Verwendung als Füllungsmaterial weisen ganz besonders bevorzugt die folgende Zusammensetzung auf:
a) 2 bis 60 Gew.-%, bevorzugt 5 bis 60 Gew.-% und besonders bevorzugt 10 bis 60 Gew.-% mindestens eines Vinylcyclopropans der allgemeinen Formel I,
b) 0,1 bis 3 Gew.-%, bevorzugt 0,2 bis 3,0 Gew.-%, besonders bevorzugt 0,2 bis 1 Gew.-% mindestens eines Initiators für die radikalische Polymerisation, vorzugsweise einen Photoinitiator,
c) 0 bis 50 Gew.-%, bevorzugt 0 bis 40 Gew.-% und besonders bevorzugt 0 bis 30 Gew.-% andere(s) Monomer(e), und
d) 10 bis 85 Gew.-%, bevorzugt 10 bis 80 Gew.-% Füllstoff (e) .

Wenn nicht anders angegeben beziehen sich alle Mengenangaben hierin auf die Gesamtmasse des Werkstoffs. Die einzelnen Mengenbereiche können separat gewählt werden.

Besonders bevorzugt sind solche Werkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind.

Dentalwerkstoffe werden offenbart, die ausschließlich Monomere enthalten, die als radikalisch polymerisierbare Gruppen Vinylcyclopropyl (VCP)-Gruppen aufweisen.

Die erfindungsgemäßen Werkstoffe eignen sich besonders als Dentalwerkstoffe, insbesondere als dentale Adhäsive, Zemente, Füllungskomposite und Verblendmaterialien sowie als Materialien zur Herstellung von Prothesen, künstlichen Zähnen, Inlays, Onlays, Kronen und Brücken. Sie zeichnen sich gegenüber auf Dimethacrylaten basierende Materialien durch einen deutlich geringeren Polymerisationsschrumpf und gegenüber bekannten polymerisierbaren Cyclopropanderivaten durch eine bessere Polymerisationsreaktivität aus, insbesondere bei der Photopolymerisation.

Die Dentalwerkstoffe eignen sich primär zur intraoralen Anwendung durch den Zahnarzt zur Restauration geschädigter Zähne (klinische Materialien), z.B. als dentale Zemente, Füllungskomposite und Verblendmaterialien. Sie können aber auch extraoral eingesetzt werden, beispielsweise bei der Herstellung oder Reparatur von Dentalrestaurationen, wie Prothesen, künstlichen Zähnen, Inlays, Onlays, Kronen und Brücken (technische Materialien). Die Werkstoffe eignen sich dabei besonders zur Herstellung von Formkörpern, beispielsweise von Dentalrestaurationen, durch generative Verfahren, insbesondere durch Stereolithographie oder 3D-Druck (vgl. A. Gebhardt, Generative Fertigungsverfahren, 3. Aufl., Carl Hanser Verlag, München 2007).

Die Erfindung wird im Folgenden anhand von Ausführungsbeispielen näher erläutert.

### Ausführungsbeispiele

### Beispiel 1

### Synthese von 1-(2-Ethylcarbamoyloxyethoxycarbonyl)-1-ethoxy-carbonyl-2-vinylcyclopropan (VCP-1)

### 1. Stufe: Synthese von 1-(2-Hydroxyethoxycarbonyl)-1-ethoxycarbonyl-2-vinylcyclopropan (VCP-OH)

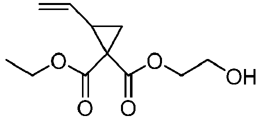

Zu einer gerührten Lösung von 1-Ethoxycarbonyl-2-vinylcyclopropancarbonsäure (36.8 g. 200 mmol), Ethylenglykol (49.7 g, 800 mmol) und 4-Dimethylaminopyridin (DMAP, 1.22 g, 1.0 mmol) in wasserfreiem Methylenchlorid (30.0 ml) wurde unter Argon und bei 0 °C Dicyclohexylcarbodiimid (DCC, 41.2 g, 200 mmol) portionsweise zugegeben. Es wurde 30 min bei 0 °C und 15 h bei RT gerührt. Das Reaktionsgemisch wurde über eine Fritte gesaugt und der Rückstand mit Methylenchlorid (3x40 ml) gewaschen. Die organische Phase wurde mit Wasser (150 ml) gewaschen und die wässrige Phase mit DCM (2 x 30 ml) extrahiert. Die vereinigten organischen Phasen wurden über wasserfreiem Natriumsulfat getrocknet. Das Lösungsmittel wurde im Vakuum abdestilliert und das Rohprodukt chromatographisch (Flash-Kieselgel mit Ethylacetat/Hexan: 1/3) gereinigt. Es wurden 32.44 g (71 % Ausbeute) einer farblosen Flüssigkeit als ein Gemisch von Diastereoisomeren (Verhältnis: Ca. 9/1) wurde erhalten.

NMR Analyse des Hauptisomers: ¹H NMR (400 MHz, CDCl₃) : δ = 1.27 (t, ³J_{HH} = 7.1 Hz, 3H, OCH₂CH₃) ; 1.60 (dd, ²J_{HH} = 5.0 Hz, ³J_{HH} = 9.0 Hz, 1H, CH₂CHCH=CH₂) ; 1.77 (dd, ²J_{HH} = 5.0 Hz, ³J_{HH} = 7.7 Hz, 1H, CH₂CHCH=CH₂) ; 2.62 (q, ³J_{HH} = 8.5 Hz, 1H, CH₂CHCH=CH₂) ; 3.82 (t, ³J_{HH} = 4.6 Hz, CH₂OH) ; 4.14-4.29 (m, 3H, CH₂OCO) ; 4.38 (dt, ²J_{HH} = 10.7 Hz, ³J_{HH} = 4.8 Hz, 1H, CH₂OCO) ; 5.13-5.20 (m, 1H, CH₂=CH); 5.27-5.37 (m, 1H, CH₂=CH); 5.41-5.53 (m, 1H, CH₂=CH). ¹³C NMR (101 MHz, CDCl₃) : δ = 14.2 (CH₃) ; 20.8 (CH₂CHCH=CH₂) ; 31.8 (CH₂CHCH=CH₂) ; 35.7 (COCCO) ; 60.9 (CH₂OH); 61.6 (CH₂OCO); 67.1 (CH₂OCO); 119.0 (CH₂=CH); 132.7 (CH₂=CH); 167.4 (C=O); 169.9 (C=O).

### 2. Stufe: Synthese von 1-(2-Ethylcarbamoyloxyethoxycarbonyl)-1-ethoxycarbonyl-2-vinylcyclopropan (VCP-1)

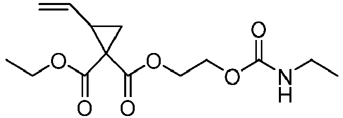

Zu einer gerührten Lösung von 1-(2-Hydroxyethoxycarbonyl)-1-ethoxycarbonyl-2-vinylcyclopropan (**VCP-OH,** 10.0 g, 43.8 mmol) und Ethylisocyanat (3.42 g, 48.2 mmol) in wasserfreiem Methylenchlorid (60.0 ml) wurde unter Argon eine Lösung von Dibutylzinndilaurat (138 mg, 0.22 mmol) in wasserfreiem Methylenchlorid (10 ml) zugegeben. Es wurde 3 h bei RT gerührt. Das Lösungsmittel wurde im Vakuum abdestilliert und Ethylacetat (150 ml) wurde dem Rückstand zugegeben. Die Lösung wurde mit 5 %iger NaOH gewaschen (2*50 ml). Die organische Phase wurde über wasserfreiem Natriumsulfat getrocknet. Das Lösungsmittel wurde im Vakuum abdestilliert. Es wurden 11.95 g (91 % Ausbeute) einer farblosen Flüssigkeit erhalten.

¹H NMR (400 MHz, CDCl₃) : δ = 1.14 (t, ³J_{HH} = 7.2 Hz, 3H, NHCH₂CH₃) ; 1.26 (t, ³J_{HH} = 7.2 Hz, 3H, OCH₂CH₃); 1.59 (dd, ²J_{HH} = 4.8 Hz, ³J_{HH} = 9.0 Hz, 1H, CH₂CHCH=CH₂) ; 1.73 (dd, ²J_{HH} = 4.8 Hz, ³J_{HH} = 7.6 Hz, 1H, CH₂CHCH=CH₂); 2.60 (q, ³J_{HH} = 8.3 Hz, 1H, CH₂CHCH=CH₂); 3.12-3.30 (m, 2H, CH₂NH); 4.08-4.44 (m, 6H, CH₂OCO) ; 4.69 (s, 1H, NH); 5.11-5.17 (m, 1H, CH₂=CH); 5.26-5.34 (m, 1H, CH₂=CH); 5.39-5.51 (m, 1H, CH₂=CH). ¹³C NMR (101 MHz, CDCl₃) : δ = 14.2 (CH₃) ; 15.2 (CH₃) ; 20.5 (CH₂CHCH=CH₂) ; 31.4 (CH₂CHCH=CH₂) ; 35.8 (COCCO); 35.9 (CH₂NH); 61.5 (CH₂OCO); 62.2 (CH₂OCO); 63.7 (CH₂OCO); 118.7 (CH₂=CH); 132.9 (CH₂=CH); 155.9 (C=O); 167.1 (C=O); 169.4 (C=O).

### Beispiel 2

### Synthese eines TCD-Diisocyanat-Di-VCP-OH-Adduktes (VCP-2)

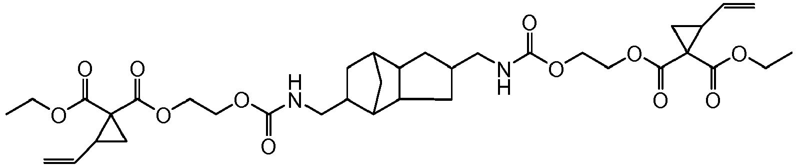

Zu einer gerührten Lösung von **VCP-OH** (9.14 g, 40.0 mmol) und Dibutylzinndilaurat (166 mg, 0.26 mmol) in wasserfreiem Methylenchlorid (70.0 mL) wurde unter Argon Bis(isocyanatomethyl)tricyclo[5.2.1.02'6]decan (TCD-Diisocyanat (Lanxess), 5.13 g, 20.8 mmol) zugegeben. Es wurde 4 h bei RT gerührt. Die Lösung wurde mit Wasser gewaschen (75 ml). Die organische Phase wurde über wasserfreiem Natriumsulfat getrocknet. Das Lösungsmittel wurde im Vakuum abdestilliert und das Rohprodukt chromatographisch (Flash-Kieselgel mit Ethylacetat/Hexan: 1/1) gereinigt. Es wurden 11.72 g (83 % Ausbeute) eines farblosen Harzes erhalten.

¹H NMR (400 MHz, CDCl₃) : δ = 0.80-2.55 (m, 24H, CH, CH₂, CH₂CHCH=CH₂, OCH₂CH₃) ; 2.60 (q, ³J_{HH} = 8.3 Hz, 2H, CH₂CHCH=CH₂) ; 2.88-3.14 (m, 4H, CH₂NH); 4.13-4.45 (m, 12H, CH₂OCO) ; 4.67-4.90 (m, 2H, NH); 5.11-5.19 (m, 2H, CH₂=CH); 5.26-5.35 (m, 2H, CH₂=CH); 5.38-5.51 (m, 2H, CH₂=CH).

### Beispiel 3 (Vergleichsbeispiel)

### Synthese von 1,8-Bis(2-vinylcyclopropan-1-carbonsäureethyl-ester-1-carbonyloxy)-3,6-dioxaoctan (VCP 5)

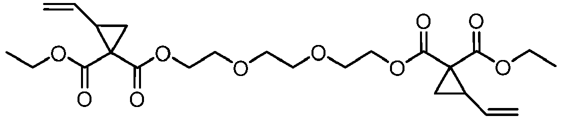

Zu einer gerührten Lösung von 1-Ethoxycarbonyl-2-vinylcyclopropancarbonsäure (12.9 g. 69.9 mmol), Triethylenglykol (5.0 g, 33.3 mmol) und DMAP (81 mg, 0.67 mmol) in wasserfreiem Methylenchlorid (40.0 ml) wurde unter Argon und bei 0 °C DCC (14.4 g, 69.9 mmol) portionsweise zugegeben. Es wurde 30 min bei 0 °C und 15 h bei RT gerührt. Das Reaktionsgemisch wurde über eine Fritte gesaugt und der Rückstand mit Methylenchlorid (3x20 ml) gewaschen. Die organische Phase wurde mit HCl 1N (75 ml), einer gesättigten NaHCO₃ Lösung (75 ml) und einer gesättigten NaCl (75 ml) Lösung gewaschen. Die organische Phase wurde über wasserfreiem Natriumsulfat getrocknet. Das Lösungsmittel wurde im Vakuum abdestilliert und das Rohprodukt chromatographisch (Flash-Kieselgel mit Ethylacetat/Heptan: 1/2) gereinigt. Es wurden 11.6 g (72 % Ausbeute) einer farblosen Flüssigkeit erhalten.

¹H NMR (400 MHz, CDCl₃) : δ = 1.26 (t, ³J_{HH} = 7.2 Hz, 6H, OCH₂CH₃); 1.57 (dd, ²J_{HH} = 4.9 Hz, ³J_{HH} = 9.0 Hz, 2H, CH₂CHCH=CH₂) ; 1.71 (dd, ²J_{HH} = 4.9 Hz, ³J_{HH} = 7.6 Hz, 2H, CH₂CHCH=CH₂) ; 2.59 (q, ³J_{HH} = 8.4 Hz, 2H, CH₂CHCH=CH₂) ; 3.62 (s, 4H, OCH₂CH₂O) ; 3.69 (t, ³J_{HH} = 4.9 Hz, 4H, OCH₂CH₂OCO) ; 4.12-4.35 (m, 8H, CH₂OCO) ; 5.10-5.16 (m, 2H, CH₂=CH); 5.25-5.33 (m, 2H, CH₂=CH); 5.37-5.49 (m, 2H, CH₂=CH). ¹³C NMR (101 MHz, CDCl₃) : δ = 14.2 (CH₃) ; 20.6 (CH₂CHCH=CH₂) ; 31.4 (CH₂CHCH=CH₂) ; 35.8 (COCCO) ; 61.5 (CH₂O) ; 64.6 (CH₂O) ; 68.9 (CH₂OCO); 70.6 (CH₂OCO) ; 118.6 (CH₂=CH) ; 133.0 (CH₂=CH); 167.2 (C=O); 169.6 (C=O).

### Beispiel 4

### Bestimmung der Reaktivität bei der Photopolymerisation

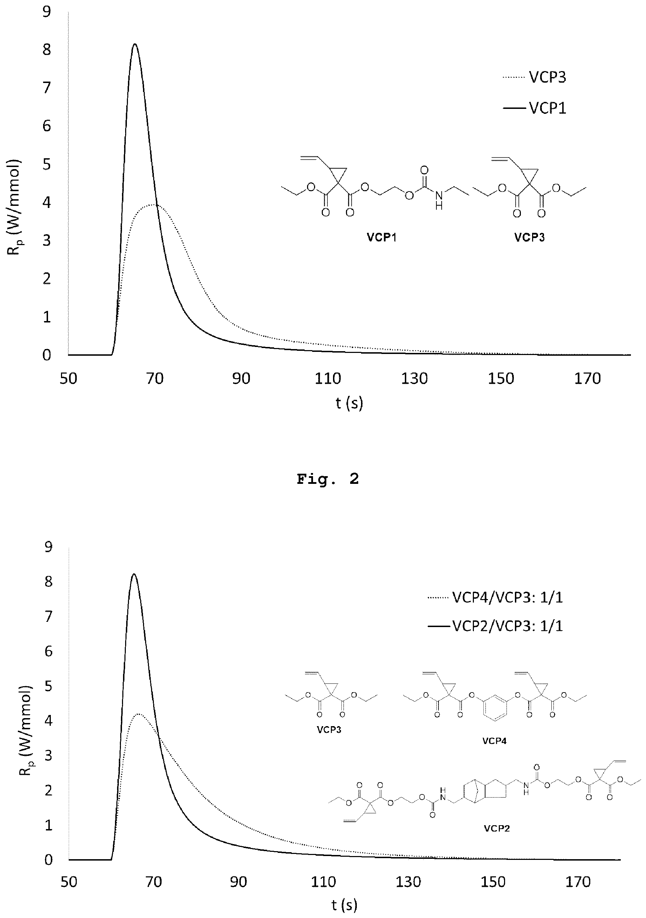

Die Reaktivität der neuen Monomeren **VCP 1** und **VCP 2** wurde im Vergleich zu den Referenzverbindungen 1,1-Bis(ethoxy-carbonyl)-2-vinylcyclopropan (**VCP 3**) bzw. Bis-(2-vinyl-1,1-dicarbonsäuremonoethylester)resorcinylester **(VCP 4)** untersucht. Zu jedem Monomer wurden 0.5 mol-% Bis(4-methoxybenzoyl)diethylgermanium (Ivocerin®, Ivoclar Vivadent AG) als Initiator zugesetzt und die Mischung in einem Differential-Scanning-Kalorimeter (Modell Diamond, Perkin Elmer) mit Photopolymerisationsaufsatz durch Bestrahlung mit einer LED-Lampe (Modell Bluephase, Ivoclar Vivadent AG) für 2 Minuten bei 37 °C polymerisiert (I = 20 mW/cm²). Die Ergebnisse sind in Figur 1 graphisch dargestellt. Das erfindungsgemäße Vinylcyclopropan **VCP 1** zeigt im Vergleich zur Referenzverbindung **VCP 3** ein deutlich höheres Maximum der Polymerisationsgeschwindigkeit.

Das deutlich höhere Maximum der Polymerisationsgeschwindigkeit zeigt, dass die erfindungsgemässen VCPs deutlich schneller polymerisieren, d.h. aushärten, als die Vergleichsverbindungen. Der entscheidende Nachteil der VCPs nach dem Stand der Technik ist ihre langsame Polymerisation.

Die Reaktivität der Mischungen **VCP 2/VCP 3** (1 mol/1 mol) und **VCP 4/VCP 3** (1 mol/1 mol) wurde anschließend unter den gleichen Bedingungen (0.5 mol% Ivocerin®, 37 °C, I = 20 mW/cm²) bestimmt. Es wurde festgestellt, dass die Mischung **VCP 2/VCP 3** deutlich reaktiver als die Referenzmischung **VCP 4/VCP 3** ist (Figur 2). Die Ergebnisse belegen, dass die Einführung von Urethangruppen zu einer signifikanten Zunahme der Reaktivität führt.

### Beispiel 5

### Herstellung von Kompositen

Komposite auf Basis einer Mischung von **VCP 2/VCP 5** (1/1: Gew./Gew.) und **TCD 1**/TEGDMA (1/1: Gew./Gew.) wurden hergestellt (Tabelle 1). Die Kompositpasten wurden mittels Kneter hergestellt. Von den Materialien wurden entsprechende Prüfkörper präpariert, die 2 mal 3 min mit einer dentalen Lichtquelle (Spectramat®, Ivoclar Vivadent AG) bestrahlt und damit ausgehärtet wurden. Nach der ISO-Norm ISO-4049 (Dentistry - Polymer-based filling, restorative and luting materials) erfolgte die Bestimmung der Biegefestigkeit und des Biege-E-Moduls (Tabelle 2). Die Messungen wurden nach 24 h Lagerung in Wasser (37 °C) durchgeführt. Der Schrumpf wurde durch Auftrieb (Archimedes-Methode) bestimmt (Tabelle 2). Das Komposit **A** auf Basis des Vinylcyclopropans gemäß Formel I weist einen deutlich geringeren Polymerisationsschrumpf als das methacrylatbasierende Komposit **B** auf.

**Tabelle 1: Zusammensetzung der hergestellten Komposite**

| **Komponente** | **Komposit A [Gew.-%]** | **Komposit B^{*)} [Gew.-%]** |
|---|---|---|
| VCP 2 | 8.70 | - |
| VCP 5 | 8.70 | - |
| TCD 1⁷⁾ | - | 8.70 |
| TEGDMA¹⁾ | - | 8.70 |
| Ivocerin®⁵⁾ (Initiator) | 0.1 | 0.1 |
| EvoCeram® Isofüller SDI⁶⁾ | 34.00 | 34.00 |
| Barium-aluminium-borosilikatglas-Füller²⁾ | 33.50 | 33.50 |
| SiO₂-ZrO₂ Sphärosil³⁾ | 10.00 | 10.00 |
| YbF₃⁴⁾ | 5.00 | 5.00 |

| | | |
|---|---|---|
| ^{*)} Vergleichsbeispiel ¹⁾ Triethylenglykoldimethacrylat ²⁾ Glaspulver GM 27884, 1 µm, silanisiert (Firma Schott) ³⁾ Firma Tokoyama Soda, ⁴⁾ Firma Auer Remy ⁵⁾ Bis(4-methoxybenzoyl)diethylgermanium ⁶⁾ Kompositfüller: Gewichtsmittlere Partikelgröße 30-40 µm 7) | | |

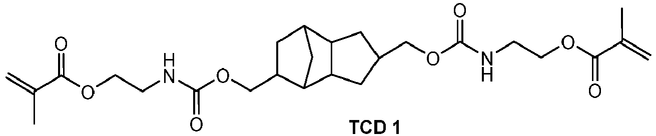

**Tabelle 2: Mechanische Eigenschaften und Volumenschrumpf**

| **Komposit** | **Biegefestigkeit (MPa)** | **Biege-E-Modul (GPa)** | **Volumenschrumpf (Vol.-%)** |
|---|---|---|---|
| A | 128.3 ± 5.0 | 7.8 ± 0.4 | 2.7 ± 0.3 |
| B^{*)} | 130.0 ± 8.9 | 10.2 ± 0.4 | 3.6 ± 0.5 |

| | | | |
|---|---|---|---|
| ^{*)} Vergleichsbeispiel | | | |

### Beispiel 6

### Herstellung von Kompositen mit verbesserten mechanischen Eigenschaften

Analog zum Beispiel 5 wurden die Kompositpasten auf Basis von **VCP 4** hergestellt (Tabelle 3). **VCP 1** und **VCP 3** wurden als Verdünner eingesetzt. Die Ergebnisse der mechanischen Eigenschaften sind in Tabelle 4 dargestellt und zeigen, dass das hochreaktive Vinylcyclopropan **VCP 1** zu einer Verbesserung der mechanischen Eigenschaften im Vergleich zu dem Vinylcyclopropan **VCP 3,** das dem Stand der Technik entspricht, führt.

**Tabelle 3 : Zusammensetzung der Komposite**

| **Komponente** | **Komposit D (Gew.-%)** | **Komposit E^{*)} (Gew.-%)** |
|---|---|---|
| VCP 1 | 5.22 | - |
| VCP 3 | - | 5.22 |
| VCP 4 | 12.18 | 12.18 |
| Ivocerin®⁵⁾ | 0.1 | 0.1 |
| EvoCeram Isofüller SDI⁶⁾ | 34.00 | 34.00 |
| Barium-aluminiumborosilikatglas-Füller²⁾ | 33.50 | 33.50 |
| SiO₂-ZrO₂ Sphärosil³⁾ | 10.00 | 10.00 |
| YbF₃⁴⁾ | 5.00 | 5.00 |

| | | |
|---|---|---|
| ^{*)} Vergleichsbeispiel ²⁻⁶⁾ wie Tabelle 1 | | |

**Tabelle 4: Mechanische Eigenschaften der Komposite**

| **Material** | **Biegefestigkeit (MPa)** | **Biege-E-Modul (GPa)** |
|---|---|---|
| D | 108.8 ± 8.8 | 7.9 ± 0.4 |
| E^{*)} | 83.3 ± 9.1 | 6.3 ± 0.6 |

| | | |
|---|---|---|
| ^{*)} Vergleichsbeispiel | | |

## Patentansprüche

1. Dentalwerkstoff, der mindestens ein Vinylcyclopropan der allgemeinen Formeln I enthält, 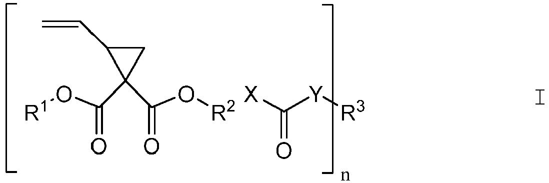 in der
die Variablen die folgenden Bedeutungen haben:
X, Y jeweils O oder NH, wobei X und Y nicht dieselbe Bedeutung haben können,
R¹ ein verzweigter oder vorzugsweise linearer C₁-C₄-Alkylrest,
R² ein verzweigter oder vorzugsweise linearer C₂-C₄-Alkylrest,
R³ ein aliphatischer linearer oder verzweigter C₁-C₂₀-Kohlenwasserstoffrest, ein aromatischer C₆-C₁₄-Rest, ein aromatischer oder nicht aromatischer heterocyclischer Rest, der 4 bis 12 Kohlenstoffatome und 1 bis 2 Heteroatome enthält, die aus N- oder O-Atomen ausgewählt sind, ein alicyclischer C₆-C₁₂-Kohlenwasserstoffrest ist, wobei es sich bei den cyclischen Resten in allen Fällen um mono- oder polycyclische Gruppen handeln kann, oder ein aliphatischaromatischer C₇-C₂₀-Rest,
n 1 oder 2.

2. Dentalwerkstoff nach Anspruch 1, der zusätzlich mindestens ein weiteres radikalisch polymerisierbares Monomer und vorzugsweise auch einen Initiator für die radikalische Polymerisation enthält.

3. Dentalwerkstoff nach Anspruch 2, der als zusätzliches Monomer mindestens eine Verbindung enthält, die aus 1,1-Di(ethoxycarbonyl)- oder 1,1-Di(methoxycarbonyl)-2-vinylcyclopropan, Bis(2-vinylcyclpropan-1-carbonsäureethyl-ester-1-carbonamido)-2,2-dimethyl-4-methylhexan, 1,8-Bis-(2-vinylcyclopropan-1-carbonsäureethylester-1-carbonyl-oxy)-3,6-dioxaoctan (VCP 5), den Estern der 1-Ethoxy-carbonyl- oder 1-Methoxycarbonyl-2-vinylcyclopropancarbon-säure mit Ethylenglycol, 1,1,1-Trimethylolpropan, 1,4-Cyclohexandiol oder Resorcin, Bis-(2-vinyl-1,1-dicarbon-säuremonoethylester)resorcinylester (VCP 4), bicyclischen Cyclopropanderivaten, 2-(Bicyclo[3.1.0]hex-1-yl)acrylsäuremethyl- oder -ethylestern oder deren Disubstitutionsprodukten in 3-Stellung, (3,3-Bis(ethoxycarbonyl)bicyclo-[3.1.0]hex-1-yl)acrylsäuremethyl- oder -ethylester ausgewählt ist.

4. Dentalwerkstoff nach Anspruch 2 oder 3, der als zusätzliches Monomer mindestens ein mono- oder polyfunktionelles (Meth)acrylsäurederivat enthält, das aus Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-GMA (ein Additionsprodukt aus Methacrylsäure und Bisphenol-A-diglycidylether), ethoxy- oder propoxyliertem Bisphenol-A-Dimethacrylat, Bisphenol-A-Dimethacrylat, 2-[4-(3-Methacryloyloxyethoxyethyl)phenyl]-2-[4-(3-methacryloyloxy-ethyl)phenyl]-propan) mit 3 Ethoxygruppen, 2,2-Bis[4-(2-(meth)acryloxypropoxy)phenyl]propan, UDMA (ein Additionsprodukt aus 2-Hydroxyethylmethacrylat (HEMA) und 2,2,4-Trimethylhexamethylendiisocyanat), TMX-UDMA (ein Additionsprodukt aus einer Mischung von HEMA und Hydroxypropylmethacrylat (HPMA) mit α,α,α',α'-Tetramethyl-m-xylylendiisocyanat (TMXDI)), Di-, Tri- oder Tetraethylen-glycoldi(meth)acrylat, Trimethylolpropantri(meth)acrylat, Pentaerythrittetra(meth)acrylat, Glycerindi- und trimethacrylat, 1,4-Butandioldi(meth)acrylat, 1,10-Decandioldi-(meth)acrylat (D₃MA), 1,12-Dodecandioldi(meth)acrylat und säuregruppenhaltigen Monomeren ausgewählt ist.

5. Dentalwerkstoff nach einem der Ansprüche 1 bis 4, der zusätzlich mindestens einen partikulären Füllstoff enthält.

6. Dentalwerkstoff nach einem der Ansprüche 1 bis 5, der zusätzlich mindestens ein Additiv enthält, das aus Lösungsmitteln, vorzugsweise Wasser, Ethanol oder eine Mischung davon, Stabilisatoren, wie z.B. Polymerisationsstabilisatoren, Aromastoffen, Farbmitteln, mikrobiociden Wirkstoffen, fluoridionenabgebenden Additiven, optischen Aufhellern, Weichmachern und/oder UV-Absorbern ausgewählt ist.

7. Dentalwerkstoff nach einem der Ansprüche 1 bis 6, der
a) 2 bis 95 Gew.-%, bevorzugt 2 bis 90 Gew.-% und besonders bevorzugt 10 bis 85 Gew.-% mindestens eines Vinylcyclopropans der allgemeinen Formel I,
b) 0,01 bis 5 Gew.-%, bevorzugt 0,1 bis 3,0 Gew.-%, besonders bevorzugt 0,2 bis 2 Gew.-% mindestens eines Initiators für die radikalische Polymerisation, vorzugsweise einen Photoinitiator,
c) 0 bis 80 Gew.-%, bevorzugt 0 bis 60 Gew.-% und besonders bevorzugt 0 bis 50 Gew.-% andere(s) Monomer(e), und ggf.
d) 0 bis 85 Gew.-%, bevorzugt 0 bis 80 Gew.-% Füllstoff(e) enthält,
jeweils bezogen auf die Gesamtmasse des Werkstoffs.

8. Dentalwerkstoff nach Anspruch 7, der 0 bis 40 Gew.-% Füllstoff(e) enthält, zur Verwendung als Beschichtungsmaterial, oder der 10-70 Gew.-% Füllstoff(e) enthält, zur Verwendung als Zement, oder der 10-85 Gew.-% Füllstoff(e) enthält, zur Verwendung als Füllungskomposit.

9. Dentalwerkstoff nach Anspruch 8 zur Verwendung als Füllungsmaterial, der
a) 2 bis 60 Gew.-%, bevorzugt 5 bis 60 Gew.-% und besonders bevorzugt 10 bis 60 Gew.-% mindestens eines Vinylcyclopropans der allgemeinen Formel I,
b) 0,1 bis 3 Gew.-%, bevorzugt 0,2 bis 3,0 Gew.-%, besonders bevorzugt 0,2 bis 1 Gew.-% mindestens eines Initiators für die radikalische Polymerisation, vorzugsweise einen Photoinitiator,
c) 0 bis 50 Gew.-%, bevorzugt 0 bis 40 Gew.-% und besonders bevorzugt 0 bis 30 Gew.-% andere(s) Monomer(e), und
d) 10 bis 85 Gew.-%, bevorzugt 10 bis 80 Gew.-% Füllstoff(e) enthält,
jeweils bezogen auf die Gesamtmasse des Werkstoffs.

10. Verwendung eines Dentalwerkstoffs nach einem der Ansprüche 1 bis 9 zur Herstellung oder Reparatur von Dentalrestaurationen, Prothesen, künstlichen Zähnen, Inlays, Onlays, Kronen oder Brücken.

11. Verwendung eines Dentalwerkstoffs nach einem der Ansprüche 1 bis 10 zur Herstellung eines Formkörpers durch ein generatives Verfahren.

12. Verwendung eines Vinylcyclopropans der Formel I zur Herstellung eines Dentalwerkstoffs.

## Claims

1. Dental material that comprises at least one vinylcyclopropane of the General Formula I, 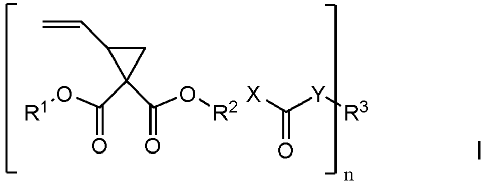 in which
the variables have the following meanings:
X, Y in each case are O or NH, wherein X and Y cannot have the same meaning,
R¹ is a branched or preferably linear C₁-C₄ alkyl group,
R² is a branched or preferably linear C₂-C₄ alkyl group,
R³ is an aliphatic linear or branched C₁-C₂₀ hydrocarbon group, an aromatic C₆-C₁₄ group, an aromatic or non-aromatic heterocyclic group that can contain 4 to 12 carbon atoms and 1 to 2 heteroatoms that are selected from N or O atoms, is an alicyclic C₆-C₁₂ hydrocarbon group, wherein the cyclic groups in all cases can be mono- or polycyclic groups, or an aliphatic-aromatic C₇-C₂₀ group,
n is 1 or 2.

2. Dental material according to claim 1 which further comprises at least one additional radically polymerisable monomer and preferably also an initiator for the radical polymerisation.

3. Dental material according to claim 2 which comprises, as the additional monomer, at least one compound that is selected from 1,1-di(ethoxycarbonyl)- or 1,1-di(methoxycarbonyl)-2-vinylcyclopropane, bis(2-vinylcyclopropane-1-carboxylic acid ethyl ester-1-carbonamido)-2,2-dimethyl-4-methylhexane, 1,8-bis(2-vinylcyclopropane-1-carboxylic acid ethyl ester-1-carbonyloxy)-3,6-dioxaoctane (VCP 5), the esters of 1-ethoxycarbonyl- or 1-methoxycarbonyl-2-vinylcyclopropanecarboxylic acid with ethylene glycol, 1,1,1-trimethylolpropane, 1,4-cyclohexanediol or resorcinol, bis-(2-vinyl-1,1-dicarboxylic acid monoethyl ester) resorcinyl ester (VCP 4), bicyclic cyclopropane derivatives, 2-(bicyclo[3.1.0]hex-1-yl)acrylic acid methyl or ethyl esters or their disubstitution products in the 3-positionf, (3,3-bis(ethoxy-carbonyl)-bicyclo-[3.1.0]-hex-1-yl)acrylic acid methyl or ethyl ester.

4. Dental material according to claim 2 or 3 which comprises, as the additional monomer, at least one mono- or polyfunctional (meth)acrylic acid derivative that is selected from methyl, ethyl, 2-hydroxyethyl, butyl, benzyl, tetrahydrofurfuryl or isobornyl (meth)acrylate, *p-*cumylphenoxyethylene glycol methacrylate (CMP-1E), bisphenol A di(meth)acrylate, bis-GMA (an addition product of methacrylic acid and bisphenol A diglycidyl ether), ethoxylated or propoxylated bisphenol A dimethacrylate, bisphenol A dimethacrylate, 2-[4-(3-methacryloyloxyethoxyethyl)phenyl]-2-[4-(3-methacryloyloxyethyl)phenyl]propane (SR-348c) with 3 ethoxy groups, 2,2-bis[4-(2-(meth)acryloxypropoxy)phenyl]propane, UDMA (an addition product of 2-hydroxyethyl methacrylate (HEMA) and 2,2,4-trimethylhexamethylene diisocyanate), TMX-UDMA (an addition product of a mixture of HEMA and hydroxypropyl methacrylate (HPMA) with α,α,α',α'-tetramethyl-*m*-xylylene diisocyanate (TMXDI)), di-, tri- or tetraethylene glycol di(meth)acrylate, trimethylolpropane tri(meth)acrylate, pentaerythritol tetra(meth)acrylate, glycerol di- and trimethacrylate, 1,4-butanediol di(meth)acrylate, 1,10-decanediol di(meth)acrylate (D₃MA), 1,12-dodecanediol di(meth)acrylate and acid-group-containing monomers.

5. Dental material according to one of claims 1 to 4 which additionally comprises at least one particulate filler.

6. Dental material according to any one of claims 1 to 5 which additionally comprises at least one additive that is selected from solvents, preferably water, ethanol or a mixture thereof, stabilisers, such as e.g. polymerisation stabilisers, flavouring agents, dyes, micro-biocidal active ingredients, fluoride-ion-releasing additives, optical brighteners, plasticisers and/or UV absorbers.

7. Dental material according to one of claims 1 to 6 which comprises
a) 2 to 95 wt %, preferably 2 to 90 wt % and particularly preferably 10 to 85 wt % of at least one vinylcyclopropane of the General Formula I,
b) 0.01 to 5 wt %, preferably 0.1 to 3.0 wt %, particularly preferably 0.2 to 2 wt % of at least one initiator for the radical polymerisation, preferably a photoinitiator,
c) 0 to 80 wt %, preferably 0 to 60 wt % and particularly preferably 0 to 50 wt % of other monomer(s), and optionally
d) 0 to 85 wt %, preferably 0 to 80 wt % filler(s),
in each case relative to the total mass of the material.

8. Dental material according to claim 7 which comprises 0 to 40 wt % filler(s), for use as a coating material, or which comprises 10-70 wt % filler(s), for use as a cement, or which comprises 10-85 wt % filler(s), for use as a filling composite.

9. Dental material according to claim 8 for use as a filling material, which comprises
a) 2 to 60 wt %, preferably 5 to 60 wt % and particularly preferably 10 to 60 wt % of at least one vinylcyclopropane of the General Formula I,
b) 0.1 to 3 wt %, preferably 0.2 to 3.0 wt %, particularly preferably 0.2 to 1 wt % of at least one initiator for the radical polymerisation, preferably a photoinitiator,
c) 0 to 50 wt %, preferably 0 to 40 wt % and particularly preferably 0 to 30 wt % of other monomer(s), and
d) 10 to 85 wt %, preferably 10 to 80 wt % filler(s),
in each case relative to the total mass of the material.

10. Use of a dental material according to one of claims 1 to 9 for the manufacture or repair of dental restorations, prostheses, artificial teeth, inlays, onlays, crowns or bridges.

11. Use of a dental material according to one of claims 1 to 10 for the manufacture of a moulded article by a generative process.

12. Use of a vinylcyclopropane of Formula I for the manufacture of a dental material.

## Revendications

1. Matériau dentaire, qui contient au moins un vinyl-cyclopropane de formule générale I : 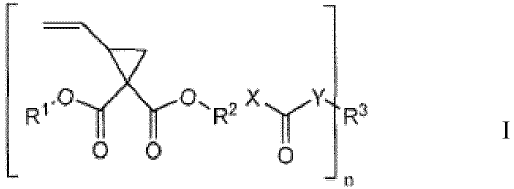 dans laquelle les symboles ont les significations suivantes :
- X et Y représentent chacun un chaînon symbolisé par O ou NH, étant entendu que X et Y ne peuvent pas avoir la même signification,
- R¹ représente un groupe alkyle en C₁-C₄, ramifié ou, de préférence, linéaire,
- R² représente un groupe alkyle en C₂-C₄, ramifié ou, de préférence, linéaire,
- R³ représente un groupe hydrocarboné aliphatique en C₁-C₂₀, linéaire ou ramifié, un groupe aromatique en C₆-C₁₄, un groupe hétérocyclique, aromatique ou non aromatique, comportant de 4 à 12 atomes de carbone et 1 ou 2 hétéroatome(s) choisi(s) parmi les atomes d'azote et d'oxygène, un groupe hydrocarboné alicyclique en C₆-C₁₂, étant entendu que les groupes cycliques peuvent, dans tous les cas, être des groupes monocycliques ou polycycliques, ou un groupe aliphatique-aromatique en C₇-C₂₀,
- et l'indice n vaut 1 ou 2.

2. Matériau dentaire conforme à la revendication 1, qui contient en outre au moins un autre monomère polymérisable par voie radicalaire, ainsi que, de préférence, un amorceur de polymérisation radicalaire.

3. Matériau dentaire conforme à la revendication 2, qui contient en tant que monomère supplémentaire au moins un composé choisi parmi les suivants : 1,1-di(éthoxy-carbonyl)-2-vinyl-cyclopropane, 1,1-di(méthoxy-carbonyl)-2-vinyl-cyclopropane, bis(2-vinyl-1-éthoxy-carbonyl-cyclopropyl-1-carbonyl-amino)-2,2,4-triméthyl-hexane, 1,8-bis(2-vinyl-1-éthoxy-carbonyl-cyclopropyl-1-carbonyl-oxy)-3,6-dioxa-octane (VCP-5), esters de l'acide 1-(éthoxy-carbonyl)-2-vinyl-cyclopropane-carboxylique ou de l'acide 1-(méthoxy-carbonyl)-2-vinyl-cyclopropane-carboxylique et de l'éthylèneglycol, du 1,1,1-triméthylol-propane, du 1,4-cyclohexane-diol ou du résorcinol, bis(2-vinyl-1-éthoxycarbonyl-1-carboxylate) de résorcinol (VCP-4), dérivés bicycliques du cyclopropane, 2-(bicyclo[3.1.0]hex-1-yl)-acrylate de méthyle ou d'éthyle, et leurs dérivés de disubstitution en position 3, (3,3-bis(éthoxy-carbonyl)-bicyclo[3.1.0]hex-1-yl)-acrylate de méthyle ou d'éthyle.

4. Matériau dentaire conforme à la revendication 2 ou 3, qui contient en tant que monomère supplémentaire au moins un dérivé mono-fonctionnel ou polyfonctionnel d'acide acrylique ou méthacrylique, choisi parmi les suivants : acrylate ou méthacrylate d'éthyle, de méthyle, de 2-hydroxy-éthyle, de butyle, de benzyle, de tétrahydrofuryle ou d'isobornyle, méthacrylate de p-cumyl-phénoxy-éthylèneglycol (CMP-1E), di(méth)acrylate de bisphénol A, bis-GMA (produit d'addition d'acide méthacrylique et d'éther diglycidylique de bisphénol A), diméthacrylate de bisphénol A éthoxylé ou propoxylé, diméthacrylate de bisphénol A, 2-[4-(3-méthacryloyloxyéthoxyéthyl)-phényl]-2-[4-(3-méthacryloyloxyéthyl)-phényl]-propane avec trois groupes éthoxy, 2,2-bis[4-(2-(méth)acryloxy-propoxy)-phényl]-propane, UDMA (produit d'addition de méthacrylate de 2-hydroxy-éthyle (HEMA) et de 2,2,4-triméthyl-hexaméthylène-diisocyanate), TMX-UDMA (produit d'addition d'un mélange HEMA/HPMA (méthacrylate d'hydroxy-propyle) et de α,α,α',α'-tétraméthyl-m-xylylène-diisocyanate (TMXDI), di(méth)acrylate de diéthylèneglycol, triéthylèneglycol ou tétraéthylèneglycol, tri(méth)acrylate de triméthylol-propane, tétra(méth)acrylate de pentaérythritol, diméthacrylate et triméthacrylate de glycérol, di-(méth)acrylate de butane-1,4-diol, di(méth)acrylate de décane-1,10-diol (DMA), di(méth)acrylate de dodécane-1,12-diol, et monomères comportant des groupes acides.

5. Matériau dentaire conforme à l'une des revendications 1 à 4, qui contient en outre au moins une charge particulaire.

6. Matériau dentaire conforme à l'une des revendications 1 à 5, qui contient en outre au moins un adjuvant, choisi parmi les suivants : solvants, de préférence de l'eau, de l'éthanol ou un mélange de ceux-ci, stabilisants, comme par exemple les stabilisants de polymérisation, arômes, colorants, microbicides, adjuvants libérant des ions fluorure, azurants optiques, plastifiants et/ou absorbeurs d'UV.

7. Matériau dentaire conforme à l'une des revendications 1 à 6, qui contient :
a) de 2 à 95 % en poids, de préférence de 2 à 90 % en poids et mieux encore de 10 à 85 % en poids d'au moins un vinyl-cyclopropane de formule générale I,
b) de 0,01 à 5 % en poids, de préférence de 0,1 à 3,0 % en poids et mieux encore de 0,2 à 2 % en poids d'au moins un amorceur de polymérisation radicalaire, de préférence un photo-amorceur,
c) de 0 à 80 % en poids, de préférence de 0 à 60 % en poids et mieux encore de 0 à 50 % en poids d'un autre ou d'autres monomère(s),
d) et en option, de 0 à 85 % en poids et de préférence de 0 à 80 % en poids de charge(s),
chaque pourcentage étant rapporté au poids total du matériau.

8. Matériau dentaire conforme à la revendication 7, qui contient de 0 à 40 % en poids de charge(s), pour utilisation en tant que matériau de revêtement, ou qui contient de 10 à 70 % en poids de charge(s), pour utilisation en tant que ciment, ou qui contient de 10 à 85 % en poids de charge(s), pour utilisation en tant que composite d'obturation.

9. Matériau dentaire conforme à la revendication 8, pour utilisation en tant que matériau d'obturation, qui contient :
a) de 2 à 60 % en poids, de préférence de 5 à 60 % en poids et mieux encore de 10 à 60 % en poids d'au moins un vinyl-cyclopropane de formule générale I,
b) de 0,1 à 3 % en poids, de préférence de 0,2 à 3,0 % en poids et mieux encore de 0,2 à 1 % en poids d'au moins un amorceur de polymérisation radicalaire, de préférence un photo-amorceur,
c) de 0 à 50 % en poids, de préférence de 0 à 40 % en poids et mieux encore de 0 à 30 % en poids d'un autre ou d'autres monomère(s),
d) et de 10 à 85 % en poids et de préférence de 10 à 80 % en poids de charge(s),
chaque pourcentage étant rapporté au poids total du matériau.

10. Utilisation d'un matériau dentaire conforme à l'une des revendications 1 à 9 pour la fabrication ou la réparation de restaurations dentaires, de prothèses, de dents artificielles, d'incrustations « inlays » ou « onlays », de couronnes ou de bridges.

11. Utilisation d'un matériau dentaire conforme à l'une des revendications 1 à 10 pour la fabrication d'une pièce par un procédé génératif.

12. Utilisation d'un vinyl-cyclopropane de formule I pour la préparation d'un matériau dentaire.
